Europäisches Patentamt

European Patent Office

Office européen des brevets

(19)

(11) Publication number: 0 359 615
A1

## (12) EUROPEAN PATENT APPLICATION

(21) Application number: 89402357.1

(22) Date of filing: 30.08.89

(51) Int. Cl.5: **A 61 L 15/60**
A 61 L 15/22

(30) Priority: 01.09.88 US 239518

(43) Date of publication of application:
21.03.90 Bulletin 90/12

(84) Designated Contracting States:
AT BE CH DE ES FR GB GR IT LI LU NL SE

(71) Applicant: JAMES RIVER CORPORATION OF VIRGINIA
Tredegar Street P.O. Box 2218
Richmond, VA 23217 (US)

(72) Inventor: Makoui, Kambiz B.
333 Winnebago Avenue
Menasha, WI 54952 (US)

Reeves, Ralph H.
4545-B West Pine
Appleton, WI 54915 (US)

Hartman, Richard R.
2528 Bruce Drive
Kalamazoo, MI 49008 (US)

Clapp, Richard T.
4760 Battenkill
Galesburg, MI 49053 (US)

(74) Representative: David, Daniel
KAYSERSBERG 54, avenue Hoche
F-75008 Paris (FR)

(54) Manufacture of superabsorbent composite structures.

(57) A method for the manufacture of a superabsorbent fibrous structure in which a dry solid absorbent is applied directly to a wet laid web of cellulosic fibers prior to drying of the wet web. A preformed web or sheet may be laid over the solid absorbent on the wet web prior to drying the resulting laminate structure.

Fig.1.

EP 0 359 615 A1

**Description**

# MANUFACTURE OF SUPERABSORBENT COMPOSITE STRUCTURES

This invention relates to a method and apparatus for the manufacture of water absorbent fibrous structures. In one of its more specific aspects, this invention relates to a method for incorporating a dry solid absorbent in a wet laid cellulosic fiber web prior to drying of the web. The resultant water absorbent fibrous structure may be used as the absorbent core for personal and health care products of various types.

Absorbent structures composed of absorbent fibrous material, such as cellulosic paper tissue, absorbent fabrics and compacted absorbent natural or synthetic fibers, alone or in combination with superabsorbent polymers, are known in the art. Such structures are useful in a variety of personal and health care products where absorption of liquid and retention of absorbed liquid are important.

By the method of this invention, a composite laminate structure comprising a cover sheet and a solid superabsorbent material on a wet base sheet may be made on a standard papermaking machine, e.g. on a fourdrinier machine. Such laminates are useful as inserts for sanitary products, such as diapers, adult incontinent pads and feminine hygiene pads. These laminates can be used also as an insert for reusable baby pants or feminine panties. The composite may comprise also a barrier-like material on one side, such as a polyethyene sheet, and coverstock, such as a polyester web, on the other side. The polyester coverstock may contain cellulosic fluff to enhance the bulk and wicking properties of the composite. The end products may be supplied in a roll form, similar to a consumer towel, perforated and used as a low cost source of baby diapers or feminine pads. Alternatively, the superabsorbent coated web may comprise one or more layers of a multi-layered superabsorbent structure. Such structures are especially useful in diapers or incontinent pads. By using several layers of such superabsorbent laminate, with each layer designed to have specific water retention properties, the ultimate size and thickness of a diaper or pad can be minimized. Composites of water resistant filter paper and superabsorbent are useful as filter media to remove water from jet fuel, gasoline or hydraulic oil.

Other uses of such laminates include agricultural purposes, such as a wrapper for the root system of trees and plants to preserve moisture around the roots during transportation. Plant seeds, such as grass, may be mixed with a powered superabsorbent and optionally with fertilizer and/or peat moss or the like to produce a laminate for use as sod, preplanted flower beds, etc.

In the prior art, many different methods are described for the manufacture of superabsorbent laminates using dry sheets. The current practice is to make this type of laminate off the paper machine where the dry sheets are sprayed with water or other liquids, such as adhesive, before the superabsorbent powder is applied onto the sheet. The added liquid helps the superabsorbent powder become affixed to the sheets, preventing migration and sifting of the powder during manufacture or transportation and also aids in lamination. Dry sheet manufacture of this type is expensive since the base sheet or top sheet requires rewetting to promote lamination and the added water must be removed by further drying to prevent mold formation. Moisture removal requires an additional drying operation which adds to the expense of the final products. Another method of manufacture of such superabsorbent laminates involves the application of wet steam to the finished composite containing superabsorbent powder.

A number of superabsorbent polymers are known in the art. U.S. Patent No. 4,600,458 to Kramer et al. for example, includes a long list of patents disclosing superabsorbent polymers useful in absorbent structures in which the superabsorbent polymers are incorporated into an absorbent fibrous web or laminate. Among the polymers disclosed for this purpose are saponified starch-polyacrylonitrile graft copolymers, crosslinked/grafted cellulose, saponified vinyl acetate-acrylic acid copolymers, starch grafted polyvinyl acetate, acrylic acid polymers, crosslinked polyethylene oxide, and the like. In the method of this patent, superabsorbent solid particles are layered between webs of fibrous material and the layered stacks of webs crimped to retain the solid absorbent in place. McFarland et al., U.S. Patent No. 4,655,757 incorporates solid particles of superabsorbent material into a coformed layer of meltblown fibers containing wood fibers for improved absorbency. Among the superabsorbents mentioned therein are those formed from hydrolyzed cross-linked polyacrylamides, polyacrylates, polymers of acrylic polymers or their copolymers. Sodium polyacrylate hydrocolloid particles are preferred as superabsorbents.

Fibrous slivers and absorbent structures having superabsorbents or hydrocolloids distributed therethrough are disclosed in U.S. Patent No. 4,340,556 to Ciencewicki and U.S. Patent No. 4,596,567 to Iskra wherein some superabsorbents are described as having a backbone of natural or synthetic polymers with hydrophilic groups, or polymers containing hydrophilic groups, chemically bonded thereto or in intimate admixture therewith. Among the superabsorbents mentioned therein are modified natural and regenerated polymers, such as polysaccharides including cellulose, and starch and regenerated cellulose which are modified by being carboxylated, phosphonoalkylated, sulfoalkylated, or phosphorylated to render them highly hydrophilic and sometimes cross-linked to render them water insoluble, all as described in U.S. Patent No. 4,105,033 to Chatterjee et al.

The two techniques disclosed in the above-mentioned patents, incorporated herein by reference, and related patents for incorporating the superabsorbent solids into the fibrous sheets, batts or slivers involve mixing or distributing the solids in or among the fibers or onto the surface of a previously formed and dried fibrous web or sheet.

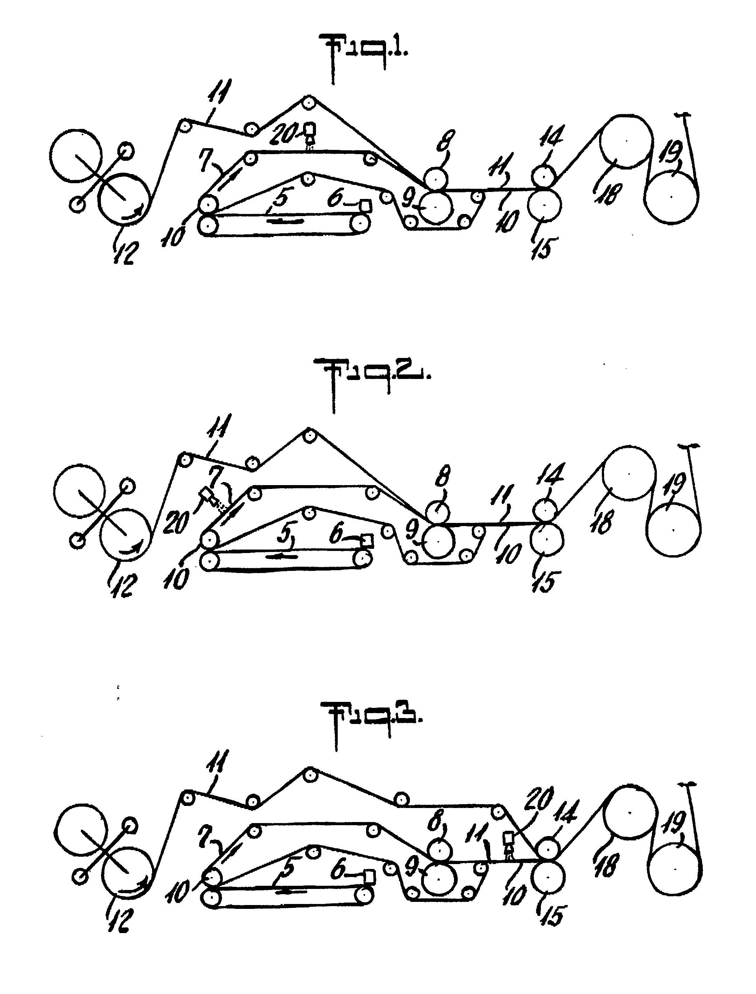

We have now found, unexpectedly, that dry solid absorbent materials of the class known as super-absorbents as referred to hereinabove may be added directly to a wet laid web prior to drying of the web. By incorporating the solid superabsorbent into an absorbent web, such as water laid paper comprising or consisting of naturally bonded or resin bonded hydrophilic cellulosic fibers, prior to drying of the web, several advantages are obtained. We have found that the solid superabsorbent is retained on the web without the need for an added tacking material ; that the wet sheet does not become slimy ; and that the web carrying the added solid superabsorbent may be dried in the usual way without disengaging the solid superabsorbent. No additional energy is required in order to fix the superabsorbent on the carrier web. The method of our invention is illustrated in the accompanying drawings wherein Figs. 1, 2 and 3 are diagrammatic representations of a fourdrinier papermaking machine illustrating the application of the method of this invention to a conventional papermaking process.

With reference to the figures wherein like numerals represent like parts, a fourdrinier papermaking machine is illustrated which comprises a forming wire 5 onto which an aqueous fiber furnish from a suitable source (not illustrated) is dispensed from headbox 6 forming a continuous water-laid web. The wet web 10 is transferred from the forming wire 5 to pick up wire or felt 7 and carried to a set of pressing rolls 8 and 9 where some of the water remaining in the web is expressed. A preformed backup web 11, suitably a dry water-laid web, a spunbonded fiber web, a lightly bonded non-woven nylon web, a water impervious sheet, or a non-woven or loosely woven fabric, is supplied from unwind roll 12 and laid on the wet web 10 carried by wire 7 at the nip of pressing rolls 8 and 9. The composite laminate web comprising webs 10 and 11 is passed through pressing rolls 14 and 15 removing more water from the laminate web and then dried on dryer drums 18 and 19. In accordance with one embodiment of the method of this invention, as illustrated in Fig. 1 of the drawings, a powder gun 20 is positioned at a mid point along the path of travel of the wet web from the forming wire 5 to the first set of pressing rolls 8 and 9. In another embodiment of the method of this invention, the powder gun 20 is positioned at a point near the pickup point of the web from the forming wire 5 to the pick up felt 7. This arrangement provides the maximum period of time of contact between the superabsorbent powder and the wet sheet prior to pressing and drying. Still another embodiment of the method of this invention is illustrated in Fig. 3 wherein the powder gun 20 is positioned immediately adjacent the nip of pressing rolls 8 and 9. This arrangement provides minimum contact time between the superabsorbent powder and the wet web prior to pressing and drying of the composite laminate web.

The selection of the position of the powder gun 20 relative to the forming wire 5 and the pressing and drying section comprising rolls 8 and 9, 14 and 15, and dryer drums 18 and 19, is determined by the nature of the base web and the properties of the superabsorbent powder. Some webs, especially the higher basis weight webs composed of wood cellulose fibers, contain relatively large amounts of water. Other webs, which may be of lower basis weight or which may comprise synthetic fibers will contain less water. The extent of water absorption of the superabsorbent powder during the manufacture of the laminate depends to a large extent on the water content of the wet base web. By selecting the point of addition of the powder to the wet base web as described herein, the operator may control the extent of wetting of the superabsorbent powder prior to lamination of the wet web with the overlaid web and drying of the composite web.

Example 1

A wet laid base sheet was made on an inclined wire, low speed paper machine, producing a 25 pound per 3000 square foot ream base sheet of 50 % northern hardwood and 50 % softwood paper pulp. The base sheet was overlaid with 40 pound per ream (3000 sg. ft.) air laid latex bonded web, marketed under the tradename Airtex by James River Corporation, to form a two layer laminate.

In this example, the above described control laminate was tested for maximum absorption capacity and retention under load of a one weight percent aqueous saline solution test fluid using a device known in the industry as an Automated Gravimetric Absorbency Tester (AGAT), also known as a Gravimetric Absorbency Tester (GAT). To perform this test, a dry specimen of known weight and size is placed on a porous plate of the tester and allowed to absorb the test fluid to its maximum capacity. The amount of liquid test fluid absorbed by the specimen is measured and recorded as grams of liquid absorbed per gram of specimen under test. The initial absorption rate in grams of liquid absorbed per gram of test specimen per second and the volume change of the specimen, i.e. the percentage expansion or contraction of the specimen which takes place due to absorption of the test liquid are determined and recorded. The test fluid is supplied to the sample from a container, which is placed over a balance through a glass porous plate. Therefore, the fluid absorbed by the sample through the porous plate is reflected by the display change in the balance and recorded by a computer. When the absorption stabilizes, and the balance gives the same reading over a 15 second interval, this portion of the test is complete. To prevent flooding of the sample with the test fluid, the porous plate height is set at a slightly negative hydrostatic tension (1.5 centimeter) over the water reservoir. This permits the sample to absorb as much fluid as it will take up (demand wetability test) without flooding the sample. In this example, the absorption capacity is measured under two different confining pressures, namely 132 $g/cm^2$ and 5 $g/cm^2$. Loaded absorbency and "no load" absorption reported in following tables are the terms used to reflect the above mentioned confining pressures, respectively.

The next property measured is the retention value at a higher hydrostatic head. In this routine, the amount of fluid that drains out of the sample once the porous plate has been raised to a specified height is measured under "no load" conditions. For the glass plates with pore size of 50 microns, the height used is 26 centimeters.

The water absorption ratio is calculated from the known weight of the sample and the measured weights of water absorbed and water retained by the sample under a 5 $g/cm^2$ confining pressure. In the following tables, the absorption and retention values are reported as grams of water per gram of sample.

The initial absorption rate reported in the following tables are reported on the basis of grams of water absorbed per gram of sample per second. The rates are multiplied by 100 for ease of comparison. The loaded absorption time, reported in seconds, represents the amount of time required to satisfy the fluid demand of the specimen under a confining pressure of 132 $g/cm^2$.

The properties of this control specimen are shown in Table 1.

Table 1

| | |
|---|---|
| Basis Weight ($g/m^2$) | 117.50 |
| Bulk (cc/g) | 7.60 |
| Loaded Absorption[1] (g/g) | 3.25 |
| No Load Absorption[2] (g/g) | 4.57 |
| Water Retained[3] (g/g) | 2.08 |
| Loaded Absorption[4] ($g/m^2$) | 382 |
| No Load Absorption[4] ($g/m^2$) | 537 |
| Initial Absorption Rate[1] (g/g/sec x 100) | 30.7 |
| Loaded Absorption Time[1] (sec) | 42 |
| Volume Expansion[5] (%) | -31 |

(1) Under compressive load of 132 $g/cm^2$ and 1.5 cm hydrostatic tension.
(2) Under compressive load of 5 $g/cm^2$ and 1.5 cm hydrostatic tension.
(3) Under compressive load of 5 $g/cm^2$ and 26 cm hydrostatic tension.
(4) Obtained by multiplying No Load Absorption (g/g) by basis weight.
(5) Negative value represents volume contraction.

Examples 2-4

Laminates were prepared as in Example 1 from a 25 pound per ream wet laid base sheet and a 40 pound per ream air laid cover sheet except that a powdered superabsorbent was added to the wet base sheet from a Nordson powder applicator immediately prior to lamination and drying as illustrated in Fig. 3. The laminates were tested for water absorbency under load (132 $g/cm^2$) and 1.5 cm hydrostatic tension and "no load" (5 $g/cm^2$) and 1.5 cm hydrostatic tension and for water retention under "no load" conditions (5 $g/cm^2$) and 26 cm hydrostatic tension. Results are reported in Table II wherein the test results are compared with those of Example I.

Table II

| | | | | EXAMPLE |
| --- | --- | --- | --- | --- |
| | 1 | 2 | 3 | 4 |
| Basis Weight (g/m²) | 117.5 | 142.2 | 142.4 | 224.3 |
| Superabsorbent Add-on (g/m²) | 0 | 24.7 | 24.9 | 106.8 |
| Bulk (cc/g) | 7.6 | 6.4 | 6.6 | 5.0 |
| Loaded Absorption[1] (g/g) | 3.25 | 5.08 | 4.87 | 10.16 |
| No Load Absorption[2] (g/g) | 4.57 | 9.63 | 9.82 | 14.24 |
| Water Retained[3] (g/g) | 2.08 | 6.82 | 6.80 | 13.09 |
| Loaded Absorption[4] (g/m²) | 382 | 722 | 693 | 2279 |
| No Load Absorption[4] (g/m²) | 537 | 1369 | 1398 | 3194 |
| Initial Absorption Rate[1] (g/g/sec x 100) | 30.7 | 28.6 | 30.7 | 22.0 |
| Loaded Absorption Time[1] (sec) | 42 | 320 | 301 | 1372 |
| Volume Expansion[5] (%) | -31 | 44 | 37 | 96 |

(1) Under compressive load of 132 g/cm² and 1.5 cm hydrostatic tension.
(2) Under compressive load of 5 g/cm² and 1.5 cm hydrostatic tension.
(3) Under compressive load of 5 g/cm² and 26 cm hydrostatic tension.
(4) Obtained by multiplying No Load Absorption (g/g) by basis weight.
(5) Negative value represents volume contraction.

Examples 5-8

A laminate was made on a papermaking machine as in Example 1 with a 25 pound per ream (3000 sg. ft.) wet laid waterleaf (50 % hardwood-50 % softwood) pulp base sheet overlaid with a 24 pound per ream preformed dry weterleaf cover sheet (Example 5).

In Examples 6 to 8, a powdered superabsorbent was uniformly distributed on the wet base sheet by means of a Nordson powder applicator prior to lamination and drying as in Examples 2-4. Results of tests conducted under the same conditions as those in Examples 1 to 4 are reported in Table III.

Table III

| | | | | EXAMPLE |
| --- | --- | --- | --- | --- |
| | 1 | 2 | 3 | 4 |
| Basis Weight (g/m²) | 71.8 | 109.6 | 109.1 | 94.4 |
| Superabsorbent Add-on (g/m²) | 0 | 37.8 | 37.3 | 22.6 |
| Bulk (cc/g) | 2.9 | 5.0 | 4.9 | 6.1 |
| Loaded Absorption[1] (g/g) | 2.70 | 5.03 | 5.04 | 2.83 |
| No Load Absorption[2] (g/g) | 3.48 | 9.46 | 9.88 | 7.21 |
| Water Retained[3] (g/g) | 2.09 | 8.00 | 8.05 | 3.88 |
| Loaded Absorption[4] (g/m²) | 194 | 551 | 550 | 267 |
| No Load Absorption[4] (g/m²) | 250 | 1037 | 1078 | 681 |
| Initial Absorption Rate[1] (g/g/sec x 100) | 20.3 | 25.3 | 26.0 | 24.6 |
| Loaded Absorption Time[1] (sec) | 47 | 314 | 324 | 140 |
| Volume Expansion[5] (%) | -27 | 74 | 86 | 58 |

(1) Under compressive load of 132 g/cm² and 1.5 cm hydrostatic tension.
(2) Under compressive load of 5 g/cm² and 1.5 cm hydrostatic tension.
(3) Under compressive load of 5 g/cm² and 26 cm hydrostatic tension.
(4) Obtained by multiplying No Load Absorption (g/g) by basis weight.
(5) Negative value represents volume contraction.

Examples 9 and 10

A laminate structure (Example 9) was produced as in Example 1 except that the base sheet was a 15 pound per ream (3000 sg. ft.) wet water-laid web overlaid with a commercial bathroom tissue. A second laminate structure (Example 10) was made up with the same base sheet and the same cover sheet in which superabsorbent powder was distributed over the wet base sheet as in Examples 2-4 and 6-8. Table IV reports the results of tests conducted under the same conditions as those of Examples 1 to 8.

5

Table IV

| | EXAMPLE | |
| --- | --- | --- |
| | 9 | 10 |
| Basis Weight (g/m²) | 43.51 | 25.2 |
| Superabsorbent Add-on (g/m²) | 0 | 81.7 |
| Bulk (cc/g) | 4.0 | 7.1 |
| Loaded Absorption[1] (g/g) | 4.45 | 10.70 |
| No Load Absorption[2] (g/g) | 5.56 | 22.26 |
| Water Retained[3] (g/g) | 3.16 | 18.40 |
| Loaded Absorption[4] (g/m²) | 194 | 1340 |
| No Load Absorption[4] (g/m²) | 242 | 2787 |
| Initial Absorption Rate[1] (g/g/sec x 100) | 38.9 | 49.8 |
| Loaded Absorption Time[1] (sec) | 31 | 388 |
| Volume Expansion[5] (%) | 81 | 140 |

(1) Under compressive load of 132 g/cm² amd 1.5 cm hydrostatic tension.

(2) Under compressive load of 5 g/cm² and 1.5 cm hydrostatic tension.

(3) Under compressive load of 5 g/cm² and 26 cm hydrostatic tension.

(4) Obtained by multiplying No Load Absorption (g/g) by basis weight.

(5) Negative value represents volume contraction.

The foregoing examples clearly demonstrate the utility of the method of this invention by effectively producing highly absorbent laminates on a conventional papermaking machine without the necessity for drying the base web before adding a solid superabsorbent to the base web.

**Claims**

1. A method for the manufacture of a superabsorbent fiber structure comprising absorbent cellulosic fibers and normally solid absorbent particles which comprises :
   a) forming a water laid web of cellulosic fibers,
   b) adding a dry solid absorbent directly to the water laid web prior to drying of the web,
   c) laying a preformed web over the absorbent, and
   d) drying the resulting solid absorbent containing laminate web.

2. A method as defined in Claim 1 wherein a water impervious film is laid on the wet water-laid web over the solid absorbent prior to drying of the web.

3. A method as defined in Claim 1 wherein the solid absorbent is added as a dry powder.

4. A method as defined in Claim 1 wherein the solid absorbent is in the form of dry fibers.

5. A method as defined in Claim 1 wherein the solid absorbent is selected from the group consisting of starch-polyacrylate copolymers, saponified starch-polyacrylonitrile graft copolymers, crosslinked grafted cellulose, saponified vinyl acetate-acrylic acid copolymers, starch grafted polyvinyl acetate, acrylic acid polymers, and cross-linked polyethylene oxide.

6. A method as defined in Claim 1 wherein the weight ratio of solid absorbent to fibers in the water laid web is in the range of from about 0.1:1 to about 4:1.

**Fig.1.**

**Fig.2.**

**Fig.3.**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | US-A-4 600 458 (T.A. KRAMER et al.) * Column 6, lines 22-51; claims * | 1-6 | A 61 L 15/60 A 61 L 15/22 |
| D,A | EP-A-0 053 928 (PERSONAL PRODUCTS CO.) * Page 8, lines 16-28; page 10, lines 1-5 * | 1-6 | |
| A | GB-A-2 060 018 (PERSONAL PRODUCTS CO.) * Page 2, lines 51-57; page 3, lines 8-14 * | 1-6 | |
| A | EP-A-0 255 654 (MIRA LANZA S.p.A.) | | |

TECHNICAL FIELDS SEARCHED (Int. Cl.5)

A 61 L

The present search report has been drawn up for all claims

| Place of search | Date of completion of the search | Examiner |
|---|---|---|
| THE HAGUE | 01-12-1989 | ESPINOSA Y CARRETERO M. |

EPO FORM 1503 03.82 (P0401)